# EUROPEAN PATENT APPLICATION

(11) **EP 4 582 103 A1**
(43) Date of publication of application: **09.07.2025**
(21) Application number: 23860512.5
(22) Date of filing: 31.08.2023
(51) Int. Cl.: A61K 45/00, A61K 31/22, A61K 31/437, A61K 31/513, A61K 31/517, A61K 31/551, A61P 27/10, A61P 43/00, A61K 9/08

(54) **alpha1 BLOCKER-CONTAINING EYEDROPS FOR INHIBITING MYOPIA PROGRESSION**

(30) Priority: 31.08.2022 JP 2022138792
(71) Applicant: Tsubota Laboratory, Inc., Tokyo 160-8582 (JP)
(72) Inventor: JEONG, Heonuk, Tokyo 160-8582 (JP); KURIHARA, Toshihide, Tokyo 160-8582 (JP); TSUBOTA, Kazuo, Tokyo 160-8582 (JP)
(74) Representative: Mewburn Ellis LLP
(86) International application number: PCT/JP2023/031931
(87) International publication number: WO 2024/048750

(57) **Abstract**

To provide novel eyedrops for suppressing myopia progression capable of readily suppressing the progression of myopia by simply being administered.

The above-described problem is solved by eyedrops for suppressing myopia progression containing an α1-blocker. The α1-blocker is preferably contained as an α1-blocker salt within a range greater than 0.001 mg/mL and up to 1.4 mg/mL. The α1-blocker includes one or two or more ingredients selected from bunazosin, prazosin, urapidil, moxisylyte, and corynanthine.

## Description

### Field of the Invention

The present invention relates to eyedrops for suppressing myopia progression containing an α1-blocker.

### BACKGROUND ART

Myopia is particularly common among Asians, especially Japanese, and the percentage of those with severe myopia of -5D or greater is also high. Further, in recent years, myopia has been on the rise worldwide and, in Japan as well, the percentage of children with a naked eyesight of less than 1.0 has been increasing year by year. Furthermore, it is also known that myopia progresses rapidly during the school-age period from seven years old to 12 years old (refer to Non-Patent Document 1).

Myopia is classified into refractive myopia, accommodative myopia (pseudomyopia), and axial myopia in accordance with a mechanism of pathogenesis, but axial myopia is the main cause of myopia progression in the school-age period. The human eye is farsighted immediately after birth, and a degree of hyperopia decreases with axial elongation during a growth period, emmetropizing upon entry into the school-age period. Axial elongation after this emmetropization leads directly to myopia, and the axial length once elongated cannot be returned to its original length. Accordingly, suppression of axial elongation during the growth period to the school-age period is considered effective for the prevention or treatment of myopia (refer to Non-Patent Document 2). Further, the eye axis is elongated not only during the growth period but also in an adult period, and axial elongation in the adult period is said to be a risk factor for various eye diseases.

Therapeutic agents for preventing myopia have been proposed, such as Rho kinase inhibitors (refer to Patent Document 1), bradykinin (refer to Patent Document 2), and crocetin (refer to Patent Document 3), for example. Further, the present inventors have proposed eyedrops for suppressing myopia containing bunazosin (Patent Document 4).

### PRIOR ART DOCUMENTS

### Non-Patent Documents

Non-Patent Document 1: Igaku no ayumi, Vol. 253, No. 2 (2015)
Non-Patent Document 2: Ophthalmology, Vol. 58, No. 6, 635-641 (2016)

### Patent Documents

Patent Document 1: WO2010/010702
Patent Document 2: Japanese Laid-Open Patent Application Publication No. 2011-144111
Patent Document 3: WO2018/212152
Patent Document 4: Japanese Patent No. 6917103

### SUMMARY OF THE INVENTION

### Problems to be Solved by the Invention

An object of the present invention is to provide novel eyedrops for suppressing myopia progression capable of suppressing the progression of myopia by a simple means.

### Means for Solving the Problems

Eyedrops for suppressing myopia progression according to the present invention comprise an α1-blocker. The α1-blocker is preferably contained as an α1-blocker salt within a range greater than 0.001 mg/mL and up to 1.4 mg/mL. Preferably, the α1-blocker includes one or two or more ingredients selected from bunazosin, prazosin, urapidil, moxisylyte, and corynanthine.

### Effect of the Invention

According to the present invention, it is possible to suppress progression of myopia by simple self-administration by a myopic person without injection by a doctor at a medical facility.

### BRIEF DESCRIPTION OF THE DRAWINGS

Fig. 1 is a photograph of a mouse used in a myopia induction experiment (-30D lens mounted on right eye and 0D on left eye).
Fig. 2 shows results of refractive power when eyedrops respectively containing bunazosin, prazosin, and urapidil as α1-blockers were administered to myopic eyes and non-myopic eyes (Experiment 1).
Fig. 3 shows results of axial length when eyedrops respectively containing bunazosin, prazosin, and urapidil as α1-blockers were administered to myopic eyes and non-myopic eyes (Experiment 1).
Fig. 4 shows the results of the refractive power when eyedrops obtained by changing a content of the α1-blockers were administered to myopic eyes and non-myopic eyes (Experiment 2).
Fig. 5 shows the results of the axial length when the eyedrops obtained by changing the content of the α1-blockers were administered to myopic eyes and non-myopic eyes (Experiment 2).

### Embodiments of the Invention

Eyedrops for suppressing myopia progression containing an α1-blocker according to the present invention will now be described. The present invention is not limited to the contents of the following embodiments and examples, and includes various modifications and applications within the scope of the gist of the present invention.

The present invention, through ongoing research on myopia progression suppression by the present inventors, provides α1-blocker-containing eyedrops capable of suppressing the progression of myopia by a simple means for self-administration by a myopic person to eyes without direct injection by a doctor at a medical facility into the eyes of the myopic person. In this application, as in the experimental examples mentioned later, eyedrops of a liquid form containing α1-blockers were administered to mouse myopic models, refractive indices and axial lengths were evaluated, and a progression suppressing effect of myopia was verified.

Eyedrops are typically liquid eyedrops (also called an ophthalmic solution) provided in a mode of being filled in an eyedrop container. Such eyedrops can be easily self-administered by a myopic person into the eyes. Examples of the α1-blocker contained in the eyedrops include bunazosin, prazosin, urapidil, moxisylyte, corynanthine, and the like, and one or two or more thereof are contained.

A content of the α1-blocker is preferably within a range greater than 0.001 mg/mL and up to 1.4 mg/mL. Note that, as described in Experiment 2 mentioned later, as an α1-blocker salt, 0.01 mg/mL or greater is particularly preferred. An application and a dosage of the eyedrops vary depending on the patient's symptoms, age, and other factors, but usually administration of approximately one to two drops each, approximately one to six times per day, is sufficient. It should be noted that an upper limit value of the content is not particularly limited, but a realistic upper limit concentration is determined by a solubility and the like of the α1-blocker salt used in this application. For example, the solubility with respect to water is 7.7 to 9.1 mg/mL for bunazosin hydrochloride, 1.4 mg/mL for prazosin hydrochloride, 25 mg/mL for urapidil hydrochloride, and approximately 5 mg/mL for doxazosin hydrochloride. Considering these values, 1.4 mg/mL is set as the upper limit value for the α1-blocker salt.

Other ingredients that may be contained in the eyedrops include pharmacologically active ingredients, physiologically active ingredients, glaucoma eyedrop ingredients, and the like, and may be contained within a range that does not impair the effects of the present invention. The eyedrops may further contain one or two or more various other ingredients and additives as necessary in accordance with usual methods, corresponding to application and form, within a range that does not impair the effects of the present invention. Examples of these ingredients and additives include various additives such as flavoring or refreshing agents, preservatives, fungicides or antibacterial agents, pH regulators, chelating agents, stabilizers, isotonizing agents, and buffering agents. By incorporating an α1-blocker into a known eye drop formulation, the effect of inhibiting myopia progression can be further enhanced.

As described above, the present invention provides eyedrops for suppressing myopia progression containing an α1-blocker, but can be represented in other invention categories, such as: (i) use of a composition containing an α1-blocker as eyedrops for suppressing myopia progression; (ii) use of a composition containing an α1-blocker for manufacture of eyedrops for suppressing myopia progression; and (iii) a myopia progression suppressing method of administering a composition containing an α1-blocker as eyedrops for suppressing progression. It should be noted that a "myopia progression suppressing effect" in this application refers to an effect of suppressing the progression of axial myopia.

### Examples

The present invention will now be described in further detail by experiments below.

### [Measurement]

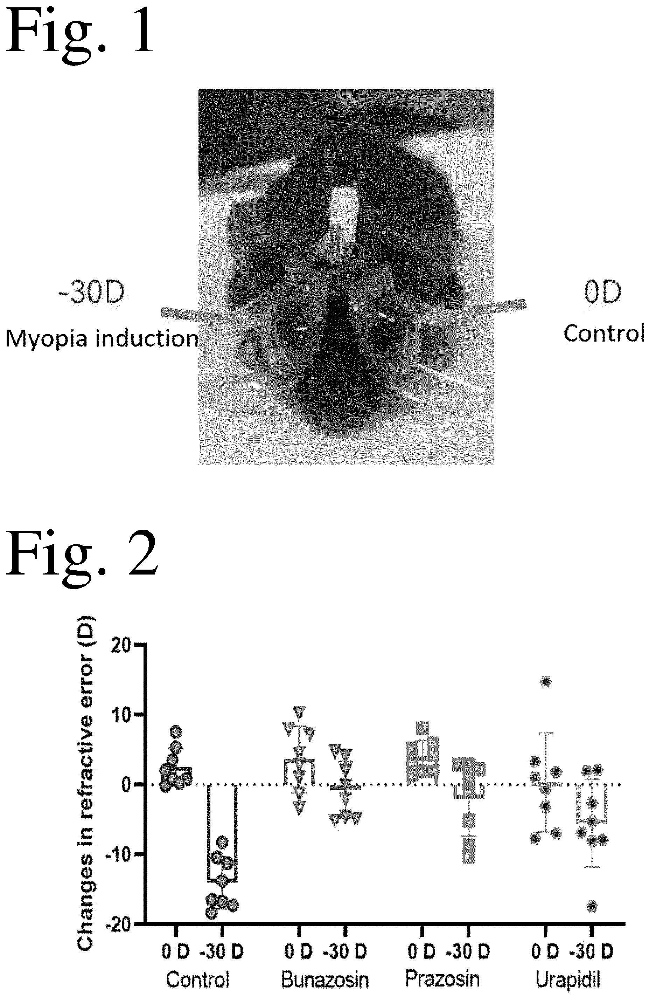

As shown in Fig. 1, a 0D (D is an abbreviation for diopter and is a unit of refractive power of a lens) lens and a -30D lens were mounted onto left eyes and right eyes, respectively, of three-week-old mice (C57BL6/J, Clea Japan, Inc.) to induce myopia over a three-week period. During the myopia induction period, eyedrops were administered once per day. Phosphate buffered saline (PBS) eyedrops were classified as a control group (Control), and α1-blocker eyedrops were classified as test groups. As the α1-blockers, bunazosin, prazosin, and urapidil were used.

The refractive index and the axial length of the mouse eyeball after three weeks were measured and the amounts of change were calculated. The refractive values were measured by using an infrared photorefractor for mice (fabricated by Prof. Schaeffel of University of Tübingen). The more negative the refractive index, the more severe the myopia. The axial lengths were measured by using spectral domain optical coherence tomography (Envisu R4310, manufactured by Leica). The greater the value of the axial length, the greater the severity of myopia.

### [Experiment 1]

### (Experiment method)

The PBS (control group), bunazosin eyedrops (bunazosin hydrochloride of 0.1 mg/mL), prazosin eyedrops (prazosin hydrochloride of 0.1 mg/mL), and urapidil eyedrops (urapidil hydrochloride of 0.1 mg/mL) were used as the eyedrops. The eyedrops were administered once per day for the three weeks of the myopia induction period.

### (Experiment results)

Fig. 2 shows the refractive index results. In the control group (PBS), the eyes with the -30D lens (-14.05 ± 3.71) became myopic as compared with the eyes with the 0D lens (2.55 ± 2.74). On the other hand, in the α1-blocker eyedrop groups, for each of bunazosin, prazosin, and urapidil, the difference between the eyes with the -30D lens (-0.74 ± 4.07, -2.03 ± 5.33, - 5.51 ± 6.29, respectively) and the eyes with the 0D lens (3.65 ± 4.72, 3.97 ± 2.34, 0.33 ± 7.07, respectively) was small, and the progression of myopia was suppressed.

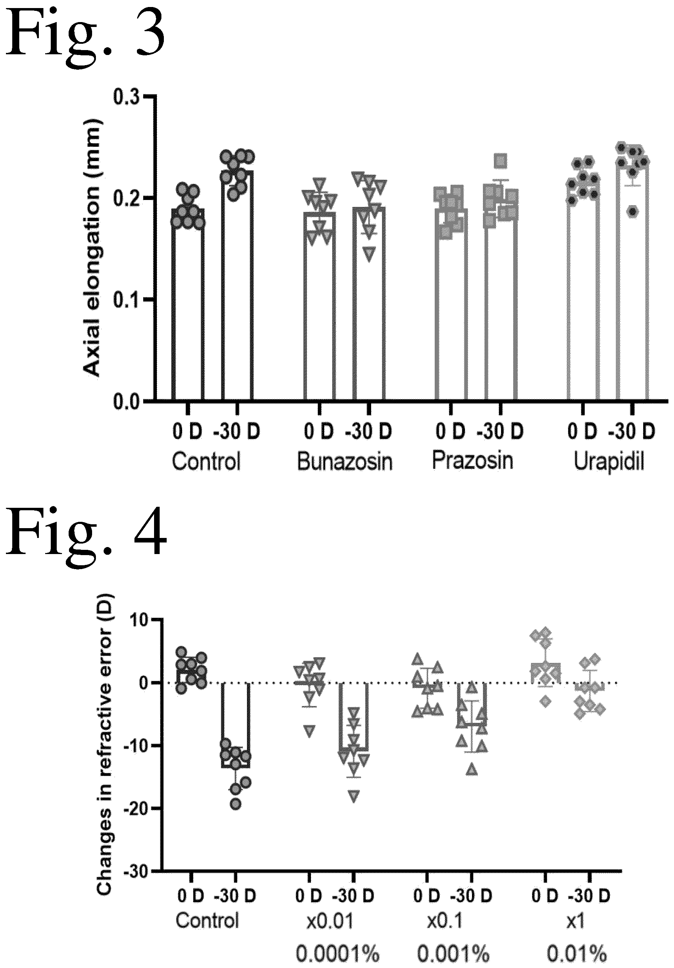

Fig. 3 shows the axial length results. In the control group (PBS), the eyes with the - 30D lens (0.227 ± 0.015) showed an elongation in axial length as compared with the eyes with the 0D lens (0.190 ± 0.014). On the other hand, in the α1-blocker eyedrop groups, for each of bunazosin, prazosin, and urapidil, the difference in axial length between the eyes with the -30D lens (0.191 ± 0.026, 0.200 ± 0.018, 0.233 ± 0.020, respectively) and the eyes with the 0D lens (0.187 ± 0.019, 0.190 ± 0.014, 0.217 ± 0.014, respectively) was small, and the progression of myopia was suppressed.

From the above results, it was confirmed that an ophthalmic solution containing an α1-blocker exhibits a myopia progression suppressing effect.

### [Experiment 2]

### (Experiment method)

α1-blocker eyedrops having concentrations (mg of α1-blocker salt contained in 1 mL: mg/mL) of 0.001 (mg/mL), 0.01 (mg/mL), and 0.1 (mg/mL) were used as the eyedrops. The eyedrops were administered once per day for the three weeks of the myopia induction period. The control group was used with PBS eyedrops not containing an α1-blocker, and the α1-blocker eyedrops were eyedrops obtained by containing bunazosin hydrochloride at the predetermined concentrations.

### (Experiment results)

Fig. 4 shows the refractive index results. With the PBS (control group, α1-blocker concentration: 0 mg/mL), the eyes with the -30D lens (-13.59 ± 3.35) became myopic as compared with the eyes with the 0D lens (2.06 ± 2.02). In the eyedrop group with an α1-blocker concentration of 0.001 (mg/mL) as well, the eyes with the -30D lens (-10.90 ± 4.13) became myopic as compared with the eyes with the 0D lens (-0.33 ± 3.47). While the myopia progression was slightly suppressed as compared with that of the PBS (control group, α1-blocker concentration: 0 mg/mL) and a slight myopia progression suppressing effect was observed, a sufficient myopia progression suppressing effect was not obtained as compared with the cases of the α1-blocker concentrations of 0.01 (mg/mL) or higher. Accordingly, it could be easily inferred that, while the α1-blocker concentration of 0.001 (mg/mL) is not sufficient, the myopia progression suppressing effect is observed as long as the α1-blocker concentration exceeds 0.001 (mg/mL). On the other hand, in the eyedrop groups with the α1-blocker concentrations of 0.01 (mg/mL) and 0.1 (mg/mL), the difference between the eyes with the -30D lens (-6.92 ± 4.08, -1.26 ± 3.28, respectively) and the eyes with the 0D lens (-0.83 ± 3.20, 3.19 ± 3.76, respectively) was small, and the progression of myopia was suppressed.

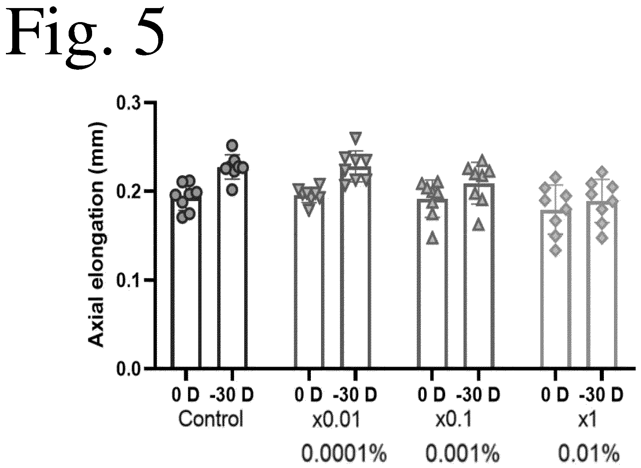

Fig. 5 shows the axial length results. With the PBS (control group, α1-blocker concentration: 0 mg/mL), the eyes with the -30D lens (0.226 ± 0.017) showed an elongation in axial length as compared with the eyes with the 0D lens (0.194 ± 0.015). In the eyedrop group with the α1-blocker concentration of 0.001 (mg/mL) as well, the eyes with the -30D lens (0.228 ± 0.017) showed an elongation in axial length as compared with the eyes with the 0D lens (0.196 ± 0.008). On the other hand, in the eyedrop groups with the α1-blocker concentrations of 0.01 (mg/mL) and 0.1 (mg/mL), there was no difference in axial length between the eyes with the -30D lens (0.209 ± 0.024, 0.189 ± 0.024, respectively) and the eyes with the 0D lens (0.192 ± 0.021, 0.180 ± 0.028, respectively), and the progression of myopia was suppressed.

From the above results, it was confirmed that an ophthalmic solution containing an α1-blocker at a predetermined concentration exhibits a myopia progression suppressing effect. It could be inferred that a myopia progression suppressing effect is observed as long as the α1-blocker concentration exceeds 0.001 (mg/mL), and a sufficient myopia progression suppressing effect was confirmed in the case of an α1-blocker concentration of 0.01 (mg/mL) or higher, in particular.

## Claims

1. Eyedrops for suppressing myopia progression comprising:
an α1-blocker.

2. The eyedrops for suppressing myopia progression according to claim 1, wherein
the α1-blocker is contained as an α1-blocker salt within a range greater than 0.001 mg/mL and up to 1.4 mg/mL.

3. The eyedrops for suppressing myopia progression according to claim 1 or 2, wherein
the α1-blocker includes one or two or more ingredients selected from bunazosin, prazosin, urapidil, moxisylyte, and corynanthine.

4. Use of a composition containing an α1-blocker as eyedrops for suppressing myopia progression.

5. Use of a composition containing an α1-blocker for manufacture of eyedrops for suppressing myopia progression.

6. A myopia progression suppressing method comprising:
administering a composition containing an α1-blocker as eyedrops for suppressing progression.
